(19) Europäisches Patentamt
European Patent Office
Office européen des brevets

(11) Publication number : **0 357 783 B1**

(12) # EUROPEAN PATENT SPECIFICATION

(45) Date of publication of patent specification : **26.10.94 Bulletin 94/43**

(51) Int. Cl.$^5$ : **G02F 1/37,** G02F 1/35, G02F 1/03, C07D 339/06

(21) Application number : **89901592.9**

(22) Date of filing : **18.01.89**

(86) International application number : **PCT/JP89/00040**

(87) International publication number : **WO 89/08863 21.09.89 Gazette 89/23**

(54) **SECONDARY NON-LINEAR OPTICAL MATERIAL AND NON-LINEAR OPTICAL ELEMENT PREPARED THEREFROM.**

(30) Priority : **09.03.88 JP 55739/88**

(43) Date of publication of application : **14.03.90 Bulletin 90/11**

(45) Publication of the grant of the patent : **26.10.94 Bulletin 94/43**

(84) Designated Contracting States : **DE FR GB NL**

(56) References cited :
**FR-A- 2 313 037**
**JP-A- 6 315 234**
**JP-A-06 329 88**
**JP-A-63 261 233**
**US-A- 3 057 875**
**US-A- 4 668 799**
**JOURNAL OF ORGANIC CHEMISTRY, vol. 30, 1965, pages 732-735, Washington, US; E.CAMPAIGNE et al.: "Dithiolium derivatives. V. 1,3-dithiol-2-ylidenes"**
**D.J.WILLIAMS Angew.Chem.96, 637-651 (1984)**

(73) Proprietor : **SUMITOMO ELECTRIC INDUSTRIES, LTD.**
**5-33, Kitahama 4-chome,**
**Chuo-ku**
**Osaka-shi, Osaka 541 (JP)**

(72) Inventor : **NOGAMI, Takashi**
**1-C39-106, Shinsenrikitamachi 3-chome**
**Toyonaka-shi**
**Osaka 565 (JP)**
Inventor : **UMEGAKI, Shinsuke 9-4-15, Chiyogaoka**
**Aso-ku**
**Kawasaki-shi**
**Kanagawa 215 (JP)**
Inventor : **UEMIYA, T. Osaka Works of Sumitomo El. Ind., Ltd.**
**1-3, Shimaya 1-chome**
**Konohama-ku**
**Osaka-shi**
**Osaka 554 (JP)**
Inventor : **SHIMIZU, Yo Osaka Works of Sumitomo El. Ind.,Ltd.**
**1-3, Shimaya 1-chome**
**Konohana-ku**
**Osaka-ki**
**Osaka 554 (JP)**
Inventor : **SHIBATA, Y. Osaka Works of Sumitomo El. Ind.,Ltd.**
**1-3, Shimaya 1-chome**
**Konohama-ku**
**Osaka-shi**
**Osaka 554 (JP)**

(74) Representative : **Hansen, Bernd, Dr.rer.nat. et al**
**Hoffmann, Eitle & Partner**
**Patentanwälte**
**Postfach 81 04 20**
**D-81904 München (DE)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a secondary nonlinear optical material and a non-linear optical element comprising the same. In particular, the present invention relates to the secondary non-linear material comprising a dithiol derivative crystal and the non-linear optical element comprising the same.

A non-linear optical effect means a phenomenon in which a polarization proportional to the higher power term than first power of an electric field of a beam occurs when the beam enters a medium. Such the non-linear optical effect often appears under the strong electric field of, for example, a laser beam. With the non-linear optical effect, secondary harmonics, Kerr effect and optical bistability are realized. In particular, the secondary optical effect which occurs in proportion to a square of the electric field of the beam is attractive since it can be applied to an element as the non-linear element such as a beam wavelength transforming element and a beam modulating element, which promises the development in the field of optoelectronics.

Nowadays, a few inorganic materials such as $KH_2PO_4$ are practically used as a material constituting such an element. However, since a non-linear optical constant of such an inorganic material is small, an extremely high voltage or an extremely intense beam is required in order to actuate the element. Therefore, a material with the larger non-linear optical constant is highly desired, and various materials have been investigated. Among the inorganic materials, lithium niobate ($LiNbO_3$) has the largest nonlinear optical constant but it has not been practically used yet since it has a disadvantage that it partly changes in a refraction index and easily destroyed when it is irradiated with an intense laser beam.

Recently, it has been found that a certain organic material has a much larger non-linear optical constant than the inorganic material. Noted is a material which has an electron donating group and an electron attracting group in a molecule as well as a $\pi$ electron system such as 2-methyl-4-nitroaniline (MNA) and which has the extremely large nonlinear optical constant. When the non-linear element comprising such an organic material is used as the beam wavelength transforming element, particularly as a secondary harmonics generating element, the element should have a wide transparent region for the use in a wide wavelength region since the wavelength of the secondary harmonics is half of that of the injected beam. Then, an electron transitional absorption band of the element is desired to reside in the shorter wavelength side. However, the conventional molecular design has such a disadvantage that the electron transitional absorption band tends to shift to the longer wavelength side since the $\pi$ electron system should be large in order to increase the secondary non-linear optical constant $\beta$ (hereinafter referred to as non-linear optical constant $\beta$) in a molecular state. Although for this reason, the material in which a benzene ring is replaced with a pyridine ring, a pyrimidine ring or a thiazole ring has been investigated, a material which has the large non-linear optical constant without the absorption in the visible light region has not been known and the element which provides the secondary non-linear optical effect, particularly an excellent one as the beam wavelength transforming element has not been produced.

JA-A-63-2988, US-A 4 668 799, FR-A 2 313 037, US-A-3 057 875, and E. Campaigne et al, J. Org. Chem. 30, 732 (1965) refer to the preparation of specific 1,3-dithiol-2-ylidene derivatives and the use thereof in pharmaceutical compositions.

D.J. Williams, Angew. Chem. 96, 637 (1984) deals with nonlinear optical properties of organic materials, but does not refer to 1,3-dithiol-2-ylidene derivatives.

The present invention has been made by taking the above problem into account. Then, it is an object to provide an organic secondary non-linear optical material which has no absorption in the visible light region, but a large non-linear optical constant, and a secondary nonlinear optical element comprising said material.

A secondary non-linear optical material according to the present invention comprises at least a compound of the general formula:

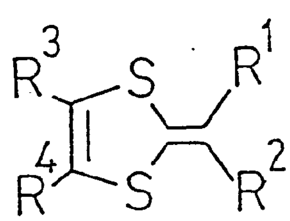

(I)

wherein $R^1$ and $R^2$ are the same or different and each represent an organic substituent, provided that at least one of $R^1$ and $R^2$ is an electron attracting group having a Hammett's substituent constant ($\sigma_{para}$) of not larger than 0.7,

and wherein $R^3$ and $R^4$ are the same or different and each represent an alkyl group, an alkoxy group, an amino

group, an alkylthio group, an aralkylthio group, an arylthio group or a group of the formula $-(CH_2)_nOH$, wherein n is a natural number,

In order to increase the non-linear optical constant β of the organic non-linear optical material, a smooth movement of an electron in a molecule is required when the electric field of the beam causes the polarization. In the compound of the general formula [I], at least one of $R^1$ and $R^2$ is the electron attracting group and the electron donating sulfur atoms are present, so that the electron can smoothly move in the molecule under the excited state. Then, the compound of the general formula [I] has the large non-linear optical constant β, whereby it provides a remarkable non-linear optical effect. In the compound of the general formula [I], the π electron system is small and the electron transitional absorption band is present in the shorter wavelength region so that the compound can be used in the wide wavelength range.

The non-linear optical element according to the present invention is characterized in that the secondary non-linear optical material comprising the compound of the general formula [I] as defined above is used in an optical waveguide portion.

The above non-linear optical element provides a remarkable non-linear optical effect since the non-linear optical material described above is used in the optical waveguide portion. Then, very intense secondary non-linear harmonics can be separated even with a weak leaser beam and an electrooptical effect can be efficiently achieved even with a low voltage change.

The secondary non-linear optical material will be hereinafter explained in detail.

In the above general formula, $R^3$ and $R^4$ are defined as an alkyl group such as methyl, ethyl, propyl, isopropyl, butyl, isobutyl, tert.-butyl, pentyl, hexyl and octyl groups; an alkoxy group such as methoxy, ethoxy, propoxy, isopropoxy, butoxy, tert.-butoxy, pentyloxy and hexyloxy groups; an amino group which may have an alkyl group such as amino, methylamino, dimethylamino, ethylamino, diethylamino, propylamino, butylamino, hexylamino and octylamino groups; an alkylthio group such as methylthio, ethylthio, propylthio, butylthio and octylthio groups; an aralkylthio or an arylthio group which may have a substituent such as benzylthio, phenylthio and p-phenylthio groups; or a group of the general formula: $-(CH_2)_n-OH$ wherein n is a natural number such as methylol, 2-hydroxyethyl and 3-hydroxypropyl groups.

In order to increase the non-linear optical constant β, the electron should be able to move smoothly when the polarization occurs by the electric field of the beam and at least one of $R^1$ and $R^2$, is the electron attracting group. As the electron attracting group, various groups can be selected and examples are a nitro group, a cyano group, a sulfonyl group which may have a halogen atom, an acyl group, a carboxyl group, an esterified carboxyl group, a carbamoyl group which may have a substituent, a sulfo group, an esterified sulfo group and a sulfamoyl group which may have a substituent. In order that the electron transitional absorption band is not present in the visible light region, the electron attracting group has a Hammett's substituent constant ($\sigma_{para}$) not larger than 0.7. In the case where the electron attracting group has the Hammett's substituent constant ($\sigma_{para}$) more than 0.7, the energy difference in between the ground state and the excited state is small so that the electron transitional absorption band may shift to the longer wavelength region (see, for example, G. B. Barlin and D. D. Perrin, Quart. Rev., 20, 75, 1966; L. M. Yaqupolskii and L. Z. Gandelsman, J. Gen. Chem., U.S.S.R., 35, 1259, 1965). In order that the electron can move smoothly in the molecule, at least one of $R^1$ and $R^2$ is the electron attracting group and further $R^1$ and $R^2$ are different groups each other in view of the molecular arrangement in the crystal. Preferably, one of $R^1$ and $R^2$ is a cyano group and the other is an esterified carboxyl group.

$R^3$ and $R^4$ are preferably the group selected from alkyl groups.

In particular, the compound of the general formula [I] wherein $R^1$ is a cyano group, $R^2$ is an alkoxycarbonyl group having 1 to 20, preferably 1 to 6 carbon atoms in the alkoxy moiety, $R^3$ is a methyl group and $R^4$ a methyl group is preferred since its non-linear optical constant β is large.

The compound of the above general formula [I] can be synthesized in a number of ways. For example, it can be produced by the ways in the below described Examples 1 and 2.

The organic non-linear optical material comprising the compound of the general formula [I] may be a single crystal of the compound of the general formula [I] or may be a mixture crystal or a mixture solid comprising the compound of the general formula [I] and other non-linear optical material, a polymer having a substituent capable of forming the hydrogen bond or a liquid crystal polymer. The compound of the general formula [I] and, for example, other nonlinear optical material may be mixed in such a composition that the properties of the compound of the general formula [I] are not affected adversely when no absorption in the visible light region is required, or they may be mixed in an arbitrary composition when the absorption in the visible light region may be present.

The single component crystal of the compound of the general formula [I] has no absorption in the visible light region, but the large non-linear optical constant β even in the crystalline state so that it provides the remarkable secondary non-linear optical effect. In the case of the mixture crystal or the mixture solid, the organic

non-linear optical material can provide the remarkable secondary nonlinear optical effect when the molecular arrangement is controlled.

As the other non-linear optical material, various organic non-linear optical materials can be used and they preferably have the large non-linear optical constant. For example, the compound having the secondary non-linear constant β larger than 5 x $10^{-30}$ esu can be used. Such an organic non-linear optical material is a compound having the electron attracting group and the electron donating group in the molecule, for example, in addition to MNA described above, a Shciff base type compound such as 1-methoxy-4-(2-nitrobenzylideneamino)benzene, 1-ethoxy-4-(2-nitrobenzylideneamino)benzene, 1-propoxy-4-(2-nitrobenzylideneanimo)benzene, 1-butoxy-4-(2-nitrobenzylideneamino)benzene, 1-hexyloxy-4-(2-nitrobenzylideneamino)benzene, 1-methoxy-4-(3-nitrobenzylideneamino)benzene, 1-ethoxy-4-(3-nitrobenzylideneamino)benzene, 1-propoxy-4-(3-nitrobenzylideneamino)benzene, 1-butoxy-4-(3-nitrobenzylideneamino)benzene, 1-hexyloxy-4-(3-nitrobenzylideneamino)benzene, 1-methoxy-4-(4-nitrobenzylideneamino)benzene, 1-ethoxy-4-(4-nitrobenzylideneamino)benzene, 1-propoxy-4-(4-nitrobenzylideneamino)benzene, 1-butoxy-4-(4-nitrobenzylideneamino)benzene, 1-pentyloxy-4-(4-nitrobenzylideneamino)benzene, 1-hexyloxy-4-(4-nitrobenzylideneamino)benzene, 1-octyloxy-4-(4-nitrobenzylideneamino)benzene, 1-nonyloxy-4-(4-nitrobenzylideneamino)benzene, 1-methyl-4-(4-nitrobenzylideneamino)benzene, 1-ethyl-4-(4-nitrobenzylideneamino)benzene, 1-propyl-4-(4-nitrobenzylideneamino)benzene, 1-butyl-4-(4-nitrobenzylideneamino)benzene, 1-(4-nitrobenzylideneamino)-4-hexylbenzene, 1-(4-nitrobenzylideneamino)-4-octylbenzene and 1-ethyl-4-(4-cyanobenzylideneamino)benzene; an anthraquinone derivative such as 1-methylaminoanthraquinone, 2-methylaminoanthraquinone, 1,4-diamino-6-ethoxycarbonylanthraquinone and 1,4-diamino-2-(1-pyrrolidinyl)anthraquinone; a styrene derivative such as β,β-dicyano-4-methoxystyrene, β,β-dicyano-4-methylstyrene and 4-dimethylamino-β,β-dicyanostyrene; 2-bromo-4-nitro-N,N-dimethylaniline; 4-N,N-dimethylamino-4′-nitrostilbene; 2-ethylamino-1,3,4-thiadiazole; a benzene derivative such as 4-(4′-dimethylanilino)-2,5,6-trifluoro-1,3-dicyanobenzene and 4-(4′-methoxythiophenoxy)-2,5,6-trifluoro-1,3-dicyanobenzene; a biphenyl derivative such as 3,5-dimethyl-2′,4′-dinitro-1,1′-biphenyl-4-ol and 3,5-di-tert.-butyl-2′,4′-dinitro-1,1′-biphenyl-4-ol; and 3-aminopyridine. The mixing ratio of the compound of the general formula [I] to the organic non-linear optical material described above is selected depending on the desired optical properties and the physical properties (for example, a melting point, a solubility and $\lambda_{max}$) of the compound.

The single crystal of the compound of the general formula [I] or the mixture crystal of the compound of the general formula [I] and the other organic non-linear optical material described above can be produced by a vapor phase growing method such as a vacuum deposition and a molecular beam eptaxial growth; a liquid phase growing method by cooling or evaporating a solution comprising the compound of the general formula [I] or by crystallization from a supersaturated solution comprising the compound; and a melt solidifying method by melting the compound of the general formula [I] and drawing up a seed crystal after contacting the melt or by crystallizing the melt of the compound in a furnace having a temperature gradient. An organic solvent used in the liquid phase growing method may be a polar solvent such as methanol and ethanol which has a hydroxyl group capable of forming the hydrogen bond, a polar solvent such as acetonitrile, ethyl acetate, diethyl ether and tetrahydrofuran or a non-polar or less-polar solvent such as benzene, toluene and cyclohexane. In the liquid phase growing method, the crystal may be produced from the solvent having an asymmetric carbon atom such as (R)-2-butanol.

The polymer having the substituent capable of forming the hydrogen bond described above is one having, for example, hydroxyl, carbonyl, carboxyl and ether groups. Polyvinyl alcohol, an acrylic resin such as polymethyl methacrylate and polyacrylate and a polyalkylene oxide such as polyethylene oxide can be exemplified.

The liquid crystal polymer described above is, for example, a side-chain type liquid crystal polymer having a mesogen group as the side chain, a side-chain type polyacrylate base liquid crystal polymer or a side chain type polysiloxane base liquid crystal polymer.

The molecular arrangement of the compound of the general formula [I] can be controlled by mixing with the polymer having a substituent capable of forming the hydrogen bond or the above liquid crystal polymer to produce the mixture solid and utilizing the molecular orientation of the polymer having the substituent capable of forming the hydrogen bond or the liquid crystal polymer. Among the compounds of the general formula [I], the compound having an alkyl moiety, especially the compound in which $R^2$ is the alkoxycarbonyl group having 1 to 20 carbon atoms in the alkoxy moiety has an excellent compatibility with the above polymers so that the molecular arrangement of the compound of the general formula [I] in the above mixture solid can be well controlled.

The mixture solid of the compound of the general formula [I] and the polymer having the substituent capable of hydrogen bonding or the liquid crystal polymer can be produced by melting the mixture of the compound of the general formula [I] and for example the liquid crystal polymer, then, gradually cooling the mixture to liquid crystal state, applying an electric field and cooling the mixture to cause solidification while applying

the electric field. By applying the electric field, the polymer having the substituent capable of forming the hydrogen bond or the liquid crystal polymer is solidified in the orientated state so that the compound of the general formula [I] can be formed which has the molecular arrangement without a symmetric center. For the mixture of the compound of the general formula [I] and the polymer having the substituent capable of forming the hydrogen bond or the liquid crystal polymer, the mixing ratio of each component is not critical as long as the compound of the general formula [I] can be in the solid state with the molecular orientation. The ratio can be selected depending on the properties (for example, phase transition temperature) of the compound of the general formula [I] and the polymer having the substituent capable of forming the hydrogen bond or the liquid crystal polymer to be used. Generally, the content of the compound of the general formula [I] is about 2 to 60 % by weight.

As described above, the organic non-linear optical material comprising the compound of the general formula [I] has the large non-linear optical constant β and remarkably achieves the non-linear optical effect. Therefore, the secondary non-linear optical material according to the present invention is suitable as the non-linear optical material used in the various elements in the field of optoelectronics, for example the material for the beam wavelength transforming element and the beam modulating element such as the phase modulating element and the amplitude modulating element. In particular, the non-linear optical material comprising the compound of the general formula [I], especially the single crystal of the compound of the general formula [I] is preferably used as the material for the beam wavelength transforming element since it has no maximum absorption in the longer wavelength region.

The non-linear optical element comprising the organic non-linear optical material described above according to the present invention will be hereinafter described in detail with reference to the accompanying drawings.

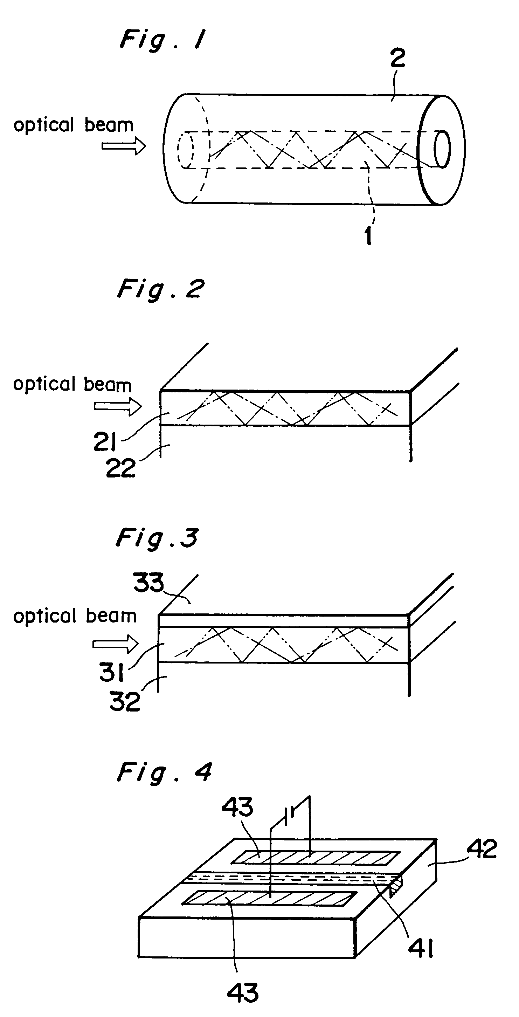

Fig. 1 shows one example of the secondary nonlinear optical element according to the present invention and schematically shows the beam wavelength transforming element of an optical waveguide type. The core (1) consisting of the organic non-linear optical material (hereinafter referred to as a non-linear medium) comprising the compound of the general formula [I], which provides the secondary non-linear optical effect, is coated with the cladding (2) comprising a medium such as glass which does not provide the secondary non-linear optical effect (hereinafter referred to as an isotropic medium). In Fig. 1, the dashed line indicates a basic wave of the injected beam and the two-dot chain line does the secondary harmonics. The beams from, for example, a laser are converged by, for example, a lens and then injected into the core (1) through one end surface of the beam wavelength transforming element. The non-linear medium constituting the core (1) provides the remarkable secondary non-linear optical effect so that the beam radiated from the other end surface of the core (1) contains the basic wave and the secondary harmonics, the latter of which can be taken out through a spectroscope means such as a prism or a filter.

Figs. 2 and 3 show two other embodiments of the beam wavelength transforming elements, respectively and the dashed line and the two-dot chain line indicate the same as in Fig. 1.

In the beam wavelength transforming element shown in Fig. 2, the optical waveguide portion (21) is formed on the substrate (22) comprising the isotropic medium. In the beam wavelength transforming element shown in Fig. 3, the optical waveguide portion (31) comprising the non-linear medium is formed between the substrate (32) comprising the isotropic medium and the top layer (33) also comprising the isotropic medium. Such the beam wavelength transforming elements are used as in the case of the beam wavelength transforming element shown in Fig. 1.

A device in the conventionally used form can be made as the beam modulating element. As one example, Fig. 4 shows a schematic view of the beam modulating element of optical waveguide type which acts longitudinally. The beam waveguiding portion (41) comprising the non-linear medium is disposed in the substrate (42) comprising the isotropic medium. Two electrodes (43) are longitudinally provided with opposed each other across the beam waveguide portion (41). Application of voltage across the electrodes (43) generates the electric field. With the element, when the beam injected through one longitudinal end surface of the beam waveguiding portion (41) is radiated from the other end surface after passing the optical waveguide portion (41), the refractive index of the non-linear medium is changed and the phase of the radiated beam is also changed. The phase modulation of the radiated beam is possible by changing the applied voltage across the electrodes (43) since the refractive index of the non-linear medium is changed by the applied voltage.

The secondary non-linear optical element according to the present invention is not limited to the above embodiments and various configurations can be conceived. For example, for the beam transforming element, only the non-linear medium can be used as the element. A possible configuration has an optical waveguide comprising the nonlinear medium formed on the substrate comprising the isotropic medium as in the above example, whereby the secondary harmonics are taken out (see, for example, J. Zyss, J. Moleaular Electronics, 1, 25 (1985)). For the beam modulating element, it may be the optical waveguide type capable of causing amplitude modulation which acts traversely or may be in the form in which the voltage is applied directly across the crystal itself. In the beam modulating element, the application direction of the electric field for the effetive

phase modulation is changed depending on, for example, the extent of the symmetry and the direction of the crystal axis of the non-linear medium. Therefore, depending on them, it is preferred to change the configuration of the electrodes adequately. With the beam wavelength transforming element of the waveguide type, it is possible to confine the beam within the waveguide so that the power density of the beam can be increased and an interaction length can be extended, which results in a high efficiency. And also phase-matching by using mode dispersion is possible.

The element can be produced in various ways. For the beam wavelength transforming elements shown in Figs. 1 to 4, the core (1) and the optical waveguide portions (21), (31) and (41) can be formed by, for example, heating a raw material for the non-linear medium in a capillary comprising the isotropic medium, on the waveguide substrate comprising the isotropic medium or between the waveguide substrates comprising the isotropic medium and then cooling it gradually, depositting the crystal on the substrate by the vacuum deposition method or radio-frequency sputtering method. Also the crystal can be deposited in the capillary, on the substrate or between the substrates from a siutable organic solvent containing the dissolved raw material for the non-linear medium. Further, after the portion which will form a contacting interface with the non-linear medium in the capillary, on the substrate or between the substrates is treated with an orientation treating agent, the beam wavelength transforming element is formed by depositing and growing the non-linear medium. The orientation treating agent is for example an inorganic salt and an organic salt (for example hexadecyltrimethylammonium bromide), a thin layer comprising a suitable polymer (for example polyamide), a metal complex or a metal thin layer (for example an obliquely deposited gold layer).

The present invention will be hereinafter described on the basis of the following examples.

## Example 1

Chloroacetaldehyde (10 millimole) was added to a mixture of disodium salt of ethyl 2-cyano-3,3-dimercaptoacrylate (10 millimole), potassium dihydrogenphosphate (10 millimole) and water (50 ml), and then stirred. After thorough cooling, a colorless precipitate was filtred and the resulted crystal was recrystallized from a mixted solvent of toluene-heptane (1 : 1). Then, the crystal (520 mg) so obtained was dissolved in concentrated sulfuric acid (10 ml), which was added in an iced water. After the precipitate was filtred and dried, it was purified with silica gel column chromatography to obtain white ethyl 1,3-dithiol-2-ylidenecyanoacetate (315 mg). An absorption in ultraviolet and visible light regions of the resulted crystal in methanol was measured to find that the maximum absorption wavelength was 368 nm.

Melting point: 178 - 179 °C

The nuclear magnetic resonance spectrum and the infrared absorption spectrum of the compound of Example 1 were measured by JOEL JNM-PMX60 NMR SPECTROMETER and HITACHI 270-30 IR SPECTROPHOTOMETER using KBr, respectively (hereinafter similarly).

1H-NMR (DMSO-$d_6$)$\delta$:

1.37 (3H, t), 4.30 (2H, q), 7.50 (2H, s)

IR ($cm^{-1}$)

2220, 1680

## Secondary non-linear optical effect of crystal

The compound obtained in Example 1 was ground in a motar, and sandwiched between a pair of preparation glasses to obtain a sample. When the sample was irradiated with a beam of YAG laser (commercially available under DCR-2 produced by Quauta-Ray Corporation), the scattering lights of the secondary harmonics (0.53 μm) were visually observed, which indicated that the compound had the secondary nonlinear optical effect. Further, the intensity of the secondary harmonics was much larger than that generated with urea.

## Evaluation of β

The electronic state of the compound obtained in Example 1 was calculated by the Pariser-Parr-Pople (PPP) method (see, for example, A. Martin, Acta. Chimica Academiae Scientiarum Hungaricae, 84, 259 (1975)). From the calculated results, the non-linear optical constant β was evaluated according to the following equation to be 20 x $10^{-30}$ esu (at 1.06 μm) (see J. L. Oudar, J. Chem. Phys. 67, 446 (1977)):

$$\beta = \frac{3e^2\hbar}{2m} \cdot \frac{w \cdot f \cdot \Delta\mu_{ge}}{[w^2 - (2\hbar\omega)^2][w^2 - (\hbar\omega)^2]}$$

wherein e is the charge of an electron, $\hbar = h/2\pi$ (wherein h is the Planck's constant), m is the mass of the electron, w is energy difference between the ground state and the excited state, $\hbar\omega$ is an energy of an injected

beam, f is oscillator intensity and $\Delta\mu_{ge}$ is moment difference of a parmanent pole between the ground state and the excited state.

The values of β for MNA and 4-N,N-dimethylamino-4'-nitrostilbene (DANS) calculated by the PPP method are shown in the table. The values correspond to those in the literature which are caluculated on the basis of measured values by J. L. Oudar et. al. according to the above equation by which the non-linear optical constant β is calculated (see J. L. Oudar, J. Chem. Phys., 67, 446 (1977)), which shows the validity to calculate the non-linear optical constant β by the PPP method. Therefore, apparently the non-linear optical constant β of the compound obtained in Example 1 is larger than that of MNA.

TABLE

| | β at 1.06 μm (x $10^{-30}$ esu) | |
|---|---|---|
| | PPP method | Reference value |
| MNA | 16.3 | 19 |
| DANS | 418 | 383 |

Example 2

Methylpropargyl bromide (10 millimole) was added in a mixture of disodium ethyl 2-cyano-3,3-dimercaptoacrylate (10 millimole) and ethanol (40 ml) and stirred for one hour at room temperature, and water (60 ml) was added in the mixture. Then, the reaction mixture was acidified with hydrochloric acid. The precipitate was filtered and dried to obtain ethyl 4-methyl-1,3-dithiol-2-ylidenecyanoacetate (1.35 g), which was recrystallized from a mixted solvent of heptane and water-methanol to give a white crystal. The maximum absorption wavelength of the crystal in methanol was 373 nm.

Melting point: 131 - 133 °C

The secondary non-linear optical constant of the crystal was measured as in Example 1 and the intense secondary harmonics were observed.

Example 3

With using the adequate starting materils, ethyl 4,5-dimethyl-1,3-dithiol-2-ylidenecyanoacetate was produced as in Example 2, of which maximum absorption wavelength in methanol was 378 nm.

Melting point: 140 - 141 °C

$^1$H-NMR ($CDCl_3$)δ:

1.37 (3H, t), 2.23 (6H, s), 4.30 (2H, q)

IR ($cm^{-1}$)

2210, 1680

The secondary non-linear optical constant of the crystal was measured as in Example 1 and the generation of the secondary harmonics with twenty-two times intense as that of urea was observed.

The crystalline structure of the compound of Example 3 was found, by X-ray structural analysis, to be the triclinic system and the PI space group, and that each molecule is arranged such that each dipole moment is not negated one another.

Example 4

With using the adequate starting materials, methy 1,3-dithiol-2-ylidenecyanoacetate was produced as in Example 1, of which maximum absorption wavelength in methanol was 368 nm.

Melting point: 204 - 205 °C

The secondary non-linear optical consatnt of the crystal was measured as in Example 1 and the intense secondary harmonics were observed.

Example 5

With using the adequate starting materials, propyl 4-methyl-1,3-dithiol-2-ylidenecyanoacetate was produced as in Example 2, of which maximum absorption wavelength in methanol was 378 nm.

Melting point: 82 - 83 °C

$^1$H-NMR ($CDCl_3$)δ:

1.00 (3H, t), 1.77 (2H, m), 2.38 (3H, s)

IR ($cm^{-1}$)

2220, 1690

The secondary non-linear optical consatnt of the crystal was measured as in Example 1 and the intense secondary harmonics were observed.

Example 6

With using the adequate starting materials, isopropyl 4-methyl-1,3-dithiol-2-ylidenecyanoacetate was produced as in Example 2, of which maximum absorption wavelength in methanol was 378 nm.

Melting point: 116 - 117°C

The secondary non-linear optical constant of the crystal was measured as in Example 1 and the intense secondary harmonics were observed.

Production of beam wavelength transforming elemetn and test results thereof

Example 7

A glass capillary having an inner diameter of about 3 μm was made from a pyrex glass tube by using an oxygen burner. Each of the compounds obtained in the above examples was melted and injected into each capillary with capillarity. Each capillary was drawn out of Bridgeman furnace kept at the temperature of 5 °C higher than the melting point of the compound to form a single crystal region of about 5 mm. Cutting out the single crystal region with a file, a YAG laser beam (1.06 μm) was injected in the single crystal in the glass capillary with the use of an objective lens of 20 magnifications. Then, green secondary harmonics (0.532 μm) were radiated from the end surface of the capillary.

Example 8

The glass capillaries were made as in Example 8. Each of the compounds obtained in the above examples 1 to 6 was heated to the boiling point of the alcohol-base solvent such as ethanol to be dissolved therein, which resulted in the saturated solution. Keeping the solution at around its boiling point, one end of the capillary heated to the boiling point was immersed in the solution so that the solution was taken into the capillary with the capillarity. Then, the both ends of the glass capillary were sealed by heating.

With a Bridgeman furnace kept at the boiling point of the solvent, the capillary was drawn out from the furnace such that the capillary was cooled from its lower end to form the single crystal region of 5 to 30 mm. After cutting out the single crystal region and polishing the end surface, the YAG laser beam (1.06 μm) was injected from one end surface of the single crystal in the glass capillary with the objective lens of 20 magnifications. Then, the green secondary harmonics (0.532 μm) were radiated from the end surface of the capillary.

Example 9

Each of the compounds obtained in the above examples 1 to 6 was heat-melted and injected in the glass capillary as in Example 7. Each capillary was placed in the Bridgeman furnace heated to the higher temperature than the boiling point of the compound. On the drawing out the glass capillary from the furnace, the single crystal of the compound grown from the alcohol solvent was attached to the cooled end surface of the glass capillary and the capillary was drawn out such that the single crystal was not melted, which resulted in the single crystal region of 1 to 30 mm in the glass capillary.

After cutting out the crystal region, the YAG laser beam (1.06 μm) was injected into the crystal in the glass capillary through one end surface. Then, the green secondary harmonics (0.523 μm) were radiated from the other end surface of each capillary.

As described above, in the compound of the general formula [I], the electron attracting substituent is bonded to the electron donating ring containing the sulfer atoms, which results in the smooth movement of the electrons when the the polarization is generated by the electric field of, for example, the beam. Therefore, the secondary non-linear optical material according to the present invention has the large non-linear optical constant β and provides the remarkable non-linear effect.

According to the non-linear optical element of the present invention, the secondary non-linear optical element comprises the above secondary non-linear optical material in the optical waveguide portion, so that the intense secondary harmonics can be separated even in the case of the weak laser beam and the electrooptic effect can be provided efficiently even when small valtage change is applied.

BRIEF DESCRIPTION OF THE DRAWINGS

Fig. 1 shows a schematical view of the beam wavelength transforming element as one example of the secondary non-linear optical element according to the present invention,
Figs. 2 and 3 show schematical views of the beam wavelength transforming element as other examples of the non-linear optical elements, respectively, and
Fig.4 shows a schematical view of the beam modulating element as other example.
(1) ... core, (2) ... cladding,
(21), (31) and (41) ... beam waveguide portion,
(22), (32) and (42) ... substrate,
(33) ... top layer, (43) ... electrode.

## Claims

1. Use of a compound of the general formula (I)

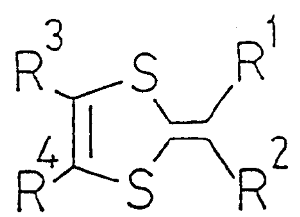

(I)

wherein $R^1$ and $R^2$ are the same or different and each represent an organic substituent, provided that at least one of $R^1$ and $R^2$ is an electron attracting group having a Hammett's substituent constant ($\sigma_{para}$) of not larger than 0.7,
and wherein $R^3$ and $R^4$ are the same or different and each represent an alkyl group, an alkoxy group, an amino group, an alkylthio group, an aralkylthio group, an arylthio group or a group of the formula $-(CH_2)_nOH$, wherein n is a natural number,
as a secondary non-linear optical material.

2. The use according to claim 1, wherein $R^1$ is a cyano group.

3. The use according to claim 1, wherein $R^2$ is an esterified carboxyl group.

4. The use according to claim 1, wherein $R^3$ is an alkyl group.

5. The use according to claim 1, wherein $R^4$ is an alkyl group.

6. The use according to claim 1, wherein $R^1$ is a cyano group, $R^2$ is an alkoxy carbonyl group which has 1 to 20 carbon atoms in the alkoxy moiety, $R^3$ is a methyl group and $R^4$ is a methyl group.

7. The use according to any of the claims 1 to 6, wherein the secondary non-linear optical material consists of single crystal of a compound of the general formula (I).

8. The use according to any of the claims 1 to 7, wherein the secondary non-linear optical material is comprised in a beam waveguide portion of a non-linear optical element.

9. The use according to claim 8, wherein the non-linear optical element is a beamwavelength transforming

element.

10. A compound of the general formula (I)

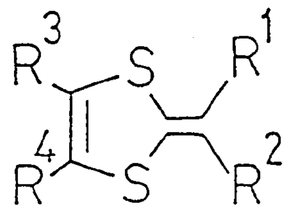

(I)

wherein $R^1$ is a cyano group, $R^2$ is an alkoxy carbonyl group which has 1 to 20 carbon atoms in the alkoxy moiety, $R^3$ is a methyl group and $R^4$ is a methyl group.

**Patentansprüche**

1. Verwendung einer Verbindung mit der allgemeinen Formel (I)

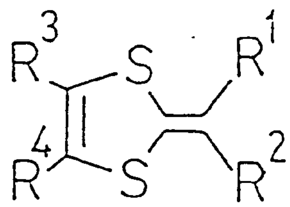

(I)

als sekundäres nichtlinear-optisches Material,
wobei $R^1$ und $R^2$ unter der Voraussetzung, daß wenigstens einer von $R^1$ oder $R^2$ eine Elektronen-anziehende Gruppe mit einer Hammett Substitutionskonstante (σ-para) von nicht mehr als 0,7 ist, gleiche oder verschiedene organische Substituenten sind, und
die Reste $R^3$ und $R^4$ gleich oder verschieden sind und jeweils eine Alkyl-, Alkoxy-, Amino-, Alkylthio-, Aralkylthio-, Arylthiogruppe oder eine Gruppe der Formel $-(CH_2)_nOH$ sind, wobei n eine natürliche Zahl ist.

2. Verwendung gemäß Anspruch 1, wobei $R^1$eine Cyanogruppe ist.

3. Verwendung gemäß Anspruch 1, wobei $R^2$einer veresterte Carboxylgruppe ist.

4. Verwendung gemäß Anspruch 1, wobei $R^3$ eine Alkylgruppe ist.

5. Verwendung gemäß Anspruch 1, wobei $R^4$ eine Alkylgruppe ist.

6. Verwendung gemäß Anspruch 1, wobei $R^1$ eine Cyanogruppe, $R^2$ eine Alkoxygruppe mit 1 bis 20 Kohlenstoffatomen im Alkoxy-Rest, $R^3$ eine Methylgruppe und $R^4$ eine Methylgruppe ist.

7. Verwendung gemäß einem der Ansprüche 1 bis 6, wobei das sekundäre linear-optische Material aus einem Einkristall der Verbindung mit der allgemeinen Formel (I) besteht.

8. Verwendung gemäß einem der Ansprüche 1 bis 7, wobei das sekundäre nichtlinear-optische Material Teil eines Lichtwellenleiters in einem nichtlinear-optischen Elements ist.

9. Verwendung gemäß Anspruch 8, wobei das optische Element ein Lichtwellenlängenumwandlungs-Element ist.

10. Verbindung mit der allgemeinen Formel (I)

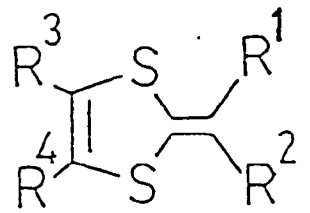

(I)

wobei $R^1$ eine Cyanogruppe, $R^2$ eine Alkoxycarbonylgruppe mit 1 bis 20 Kohlenstoffatomen im Alkoxy-Rest, $R^3$ eine Methylgruppe und $R^4$ eine Methylgruppe ist.

## Revendications

1. Utilisation d'un composé de formule générale (I) :

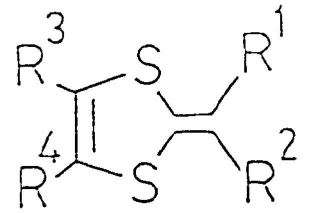

(I)

dans laquelle $R^1$ et $R^2$ sont identiques ou différents et représentent chacun un substituant organique, à condition qu'au moins l'un des groupes $R^1$ et $R^2$ soit un groupe attracteur d'électrons ayant une constante de substituant de Hammett ($\sigma_{para}$) non supérieure à 0,7, et dans laquelle $R^3$ et $R^4$ sont identiques ou différents et représentent chacun un groupe alkyle, un groupe alcoxy, un groupe amino, un groupe alkylthio, un groupe aralkylthio, un groupe arylthio ou un groupe de formule $-(CH_2)_nOH$ dans laquelle n est un nombre naturel, comme matériau optique non linéaire secondaire.

2. Utilisation selon la revendication 1, dans laquelle $R^1$ est un groupe cyano.

3. Utilisation selon la revendication 1, dans laquelle $R^2$ est un groupe carboxyle estérifié.

4. Utilisation selon la revendication 1, dans laquelle $R^3$ est un groupe alkyle.

5. Utilisation selon la revendication 1, dans laquelle $R^4$ est un groupe alkyle.

6. Utilisation selon la revendication 1, dans laquelle $R^1$ est un groupe cyano, $R^2$ est un groupe alcoxycarbonyle qui a 1 à 20 atomes de carbone dans la partie alcoxy, $R^3$ est un groupe méthyle et $R^4$ est un groupe méthyle.

7. Utilisation selon l'une quelconque des revendications 1 à 6, dans laquelle le matériau optique non linéaire secondaire consiste en un monocristal d'un composé de formule générale (I).

8. Utilisation selon l'une quelconque des revendications 1 à 7, dans laquelle le matériau optique non linéaire secondaire est compris dans une partie de guide d'ondes de faisceau d'un élément optique non linéaire.

9. Utilisation selon la revendication 8, dans laquelle l'élément optique non linéaire est un élément transformant les longueurs d'ondes de faisceau.

10. Composé de formule générale (I)

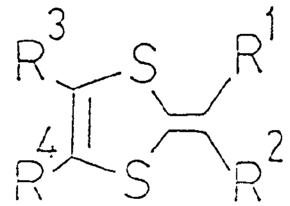

(I)

dans laquelle $R^1$ est un groupe cyano, $R^2$ est un groupe alcoxycarbonyle qui a 1 à 20 atomes de carbone dans la partie alcoxy, $R^3$ est un groupe méthyle et $R^4$ est un groupe méthyle.

*Fig. 1*

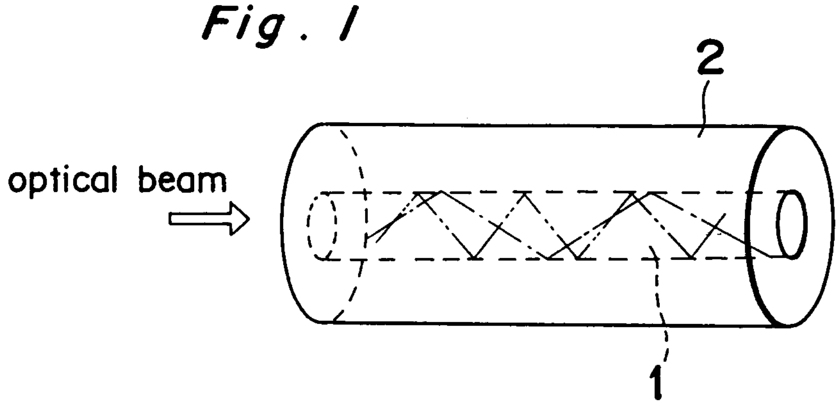


*Fig. 2*

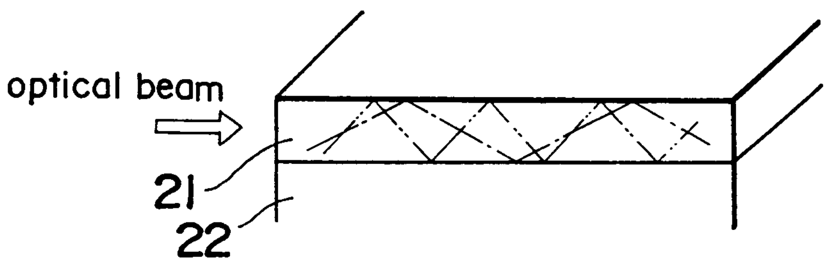


*Fig. 3*

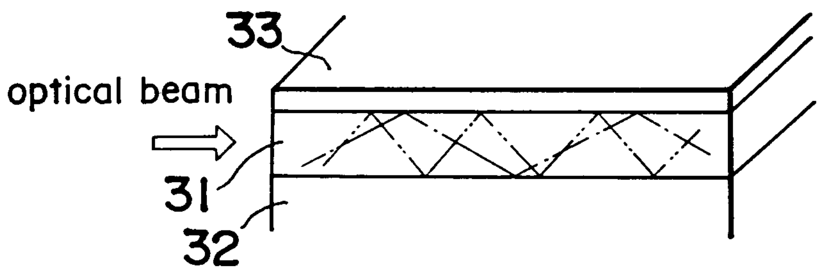


*Fig. 4*